# EUROPEAN PATENT SPECIFICATION

(11) **EP 1 787 626 B1**
(45) Date of publication and mention of the grant of the patent: **16.10.2013**
(21) Application number: 05782615.8
(22) Date of filing: 11.08.2005
(51) Int. Cl.: A61L 24/00, A61K 6/033, A61K 6/06

(54) **INJECTABLE, SELF-SETTING CALCIUM PHOSPHATE FOAM**
INJIZIERBARER SELBSTSETZENDER CALCIUMPHOSPHAT-SCHAUM
MOUSSE DE PHOSPHATE DE CALCIUM INJECTABLE À PRISE AUTOMATIQUE

(30) Priority: 12.08.2004 ES 200402045
(43) Date of publication of application: 23.05.2007
(73) Proprietor: Universitat Politècnica de Catalunya, 08034 Barcelona (ES)
(72) Inventor: GINEBRA MOLINS, Maria Pau, E-08034 Barcelona (ES); PLANELL ESTANY, Josep Antoni, E-08034 Barcelona (ES); GIL MUR, Francesc Xavier, E-08034 Barcelona (ES)
(74) Representative: ZBM Patents
(86) International application number: PCT/ES2005/070119
(87) International publication number: WO 2006/030054

(56) References cited:
- WO-A-2004/000374
- CN-A- 1 193 614
- US-A1- 2002 055 143
- US-B1- 6 642 285
- SARDA, NILSSON, BALCELLS, FERNANDEZ: "Influence of surfactant molecules as air-entraining agent for bone cement macroporosity" JOURNAL OF BIOMEDICAL MATERIALS RESEARCH PART A, vol. 65A, no. 2, 2003, pages 215-221, XP002546856
- SARDA S. ET AL: 'Influence of air-entraining agent on bone cement macroporosity' KEY ENGINEERING MATERIALS vol. 218-220, 2002, pages 335 - 338, XP008137554
- SARDA S. ET AL: 'Influence of surfactant molecules as air-entraining agent for bone cement macroporosity' JOURNAL OF BIOMEDICAL MATERIALS RESEARCH, PART A vol. 65A, no. 2, 2003, pages 215 - 221, XP002546856

## Description

### Technical field

The present invention relates to biomaterials for bone surgery and odontology, bone regeneration, bone defect fillings, stabilizing bone fractures, coating of prostheses or implants, fixing of prostheses or implants, drug delivery systems, and tissue engineering scaffolds. The present invention also relates to processes for the preparation of said biomaterials and to a kit for their obtention.

### Background

This invention is set in the area of biomaterials for bone tissue regeneration and, more specifically, in the area of calcium phosphate cements. Since 1985, when Brown and Chow submitted the first patent for this type of materials (US4518430), different formulations of calcium phosphate based cements have been developed. This type of materials is based in the mixture of one or more calcium salts with an aqueous solution to obtain a cement that is capable of setting in physiological conditions. The reaction product is a calcium phosphate with a composition very similar to the mineral phase forming the bone tissue. During setting, the plastic paste forms a solid body.

The setting of the cement is a result of the dissolution process of the reagents and the precipitation of a new phase, which occurs at room- or body-temperature. In most of the cements developed during the last years, the reaction product is a hydroxyapatite that is very similar to the biological one: it is nanocrystalline, non-stoichiometric and it can incorporate different ions depending on the composition of the reagents and the medium.

The development of cements, especially for treating bone defects, is associated with two important advantages in comparison with the use of calcium phosphates in granules or blocks. On one hand, the injectability permits implanting of the cement via minimally invasive surgical techniques. On the other hand the cement adapts perfectly to the geometry of the defect, even in complicated ones, which secure a perfect apposition bone tissue-material. This allows the stabilization of the defect and the healing process is therefore faster.

The setting properties and the hardening of the cement can be adjusted by modifying different processing parameters, such as the chemical composition of the reagents, the particle size, the addition of seed materials, etc. This makes calcium phosphate cements are very versatile materials, since they are able to adapt to clinical requirements for different applications.

Different studies have shown that the calcium phosphate cements are extremely biocompatible, osteroconductive and that they stimulate bone regeneration. However, besides being osteoconductive, the ideal cement should be capable of being resorbed with the same velocity as the bone tissue can grow with, being progressively replaced by the newly formed bone tissue. Even though the majority of the developed cements are more resorbable than the high temperature sintered apatites, they present an excessively slow resorption kinetics and in many cases the cement remains intact in the surrounding bone tissue during years.

Within the apatitic cements, which reabsorb mostly by active cellular resorption processes, the macroporosity plays an important role. These active resorption processes are based in the fact that the osteoclasts (bone cells) degrade the material layer by layer starting with the exterior bone-cement interface and going towards the interior. This process is very slow when it comes to a dense material or a microporous one. Indeed, different studies established that for the colonization process of the bone tissue to be possible, it is necessary to have pores bigger than 70µm. The conventional calcium phosphate cements, although micro- and nano- porous after the setting, do not contain macroporosity in this range that stimulates bone growth. This obstructs its resorption and its transformation into bone tissue in an adequate time period. On the contrary, if a material with an interconnected macroporosity is obtained, the angiogenesis and the tissue growth inside the material are permitted. If the macrophages and the osteoclasts can penetrate towards the interior of the material, the resorption will occur in volume and therefore it will be faster. On the other hand, the tissue ingrowth can be stimulated by filling the pores with osteoinductive or osteoconductive factors such as the morphogenic proteins (BMPs). These factors are well known to a person skilled in the art. Other therapeutic agents as antibiotics or other drugs can also be introduced in the material, mixing them with the liquid phase or the powder phase.

Different methods have been developed to obtain porosity in calcium phosphate cements. Chow et al. (US Pat.5,525,148) suggest to generate the porosity via the incorporation of a solid phase insoluble in the cement. This solid phase can be eliminated via dissolution in the physiological fluids, dissolution in different solvents or via sinterization once the cement has hardened. The porogenic agents that are proposed are sugar, sodium bicarbonate or phosphate salts.

Constantz et al. (US Pat. 5,820,632) suggest the generation of a porous structure via the addition of a soluble phase that must be eliminated later. Specifically, the addition of sodium chloride and sodium- or potassium-hydroxide is being suggested. These compounds are soluble in water and can be liberated to the physiological medium, forming porosity inside the cement.

Hirano et al. (JP 5,023,387) suggest the incorporation of a biodegradable polymer, more specifically polylactic acid, which degrades in quite a short time period, once the cement is implanted and set.

However, in the cases mentioned above, to obtain a interconnected porosity, it is necessary to use a big quantity of the porogenic agent (about 50 Vol%). This affects very significantly the properties of the material.

Bohner (EP 1150722, US Pat 6,642,285) developed porous implants based on calcium phosphate cements mixed with a hydrophobic liquid. The cement paste is mixed with the hydrophobic liquid, obtaining an emulsion. A surface active agent is suggested to be used as an additive, but only with the purpose of lowering the surface tension and facilitating the formation of the emulsion between the two phases (the cement paste and the hydrophobic liquid). Choosing adequately the hydrophobic liquid and the corresponding proportions, the setting of the cement gives a macroporous hydroxyapatite structure.

Edwards et al. (US Pat. 6,670,293) suggest a method to obtain porous cement, in which the porosity is being produced via the mixture of a calcium- and a phosphate- source, with a carbonate- source, in the powder phase. This solid phase is mixed with the liquid phase, which is an aqueous solution with an acidic component. The acid and the carbonate react, forming carbon dioxide, which produces an interconnected porosity. This method requires only a small proportion of acidic and basic components in the cement.

In S. Sarda, et al. "Influence of surfactant molecules as air-entraining agent for bone cement macroporosity" Journal of Biomedical Materials Research Part A, vol. 65A, no. 2, 2003. pages 215-221, a self setting calcium phosphate bone cement (CPC) which sets after mixing an aqueous phase and a solid phase based on calcium phosphate is disclosed. This CPC comprises an anionic surfactant agent, namely sodium dodecyl sulphate (SDS), acting as air-entraining agent. It is disclosed that during the mixing of the two phases in order to prepare the CPC, bubbles are formed and these are stabilized by the incorporation of surfactant agent.

When calcium phosphate cements form set, they form a mesh of calcium phosphate crystals, formed by precipitation from the reagents solution. This structure has a very high porosity, within the micro or nanometric range, that may reach values up to a 60%. Nevertheless, this micro or nanoporosity is not enough to allow either the growth of the bone tissue towards the interior of the material, or the angiogenesis, being this aspect crucial in order to obtain real bone regeneration. This regeneration demand the progressive colonization of the material by newly formed tissue, and the simultaneous reabsorption of the material by the action of the osteoclastic cells.

### Summary of the invention

This invention relates to foams of calcium phosphate cements that set after mixing one powder phase, consisting of one or more components like a calcium and phosphate source, with an aqueous phase. Specifically, this invention describes the adequate composition and the process to obtain foams based on calcium phosphate cements. These foams can be injected and set in the human body, giving a macroporous structure of calcium phosphate, in many cases hydroxyapatite, with a high macroporosity, produced during the foaming process. The macroporosity is superimposed to the inherent micro/nanoporosity of the calcium phosphate cements.

The object of this invention is to propose a new method to obtain macroporous calcium phosphate cements, which allow for the fast bone ingrowth and that have an adequate resorption rate. This makes possible the progressive substitution of the material by newly formed bone tissue.

A first aspect of the invention relates to a process for the preparation of a self setting calcium phosphate foam, characterized in that it comprises the mixture of two phases:
a) a liquid phase consisting of one or two aqueous solutions, one of them comprising at least one biocompatible surfactant agent responsible for the foam creation, and
b) a solid phase in powder form, comprising one or more compounds as sources of calcium and phosphate, capable of reacting with the water of the liquid phase to form a cement,
wherein the liquid phase is mixed after being submitted to a foaming process, or the foaming process is performed after the mixture and wherein the relation between the liquid phase and the solid phase is superior to 0.25 ml/g.

Another aspect of the invention relates to a self setting calcium phosphate foam obtainable by the process disclosed above.

Another aspect of the invention relates to a solid structure obtainable by setting of the foam of the present invention, having a total porosity comprised between 25 and 90 vol % and a macroporosity comprised between 2 and 80 vol %.

The calcium phosphate foams developed can be used either by injecting them directly into the body, as bone regeneration materials, or by manufacturing pre-set foams, which can be implanted in a solid state or which can be used as scaffolds or substrates for tissue engineering.

Accordingly, another aspect of the invention relates to the use of the foam of the present invention for the manufacture of a solid structure for bone regeneration, both in putty or preset state, or for the manufacture of a scaffold or substrate for tissue engineering.

It is also disclosed a kit for the preparation of the self setting calcium phosphate foam of the present invention, characterized in that comprises:
a) a liquid phase consisting of one or two aqueous solutions, one of them comprising at least one biocompatible surfactant agent responsible for the foam creation, and.
b) a solid phase in powder form, comprising one or more compounds as sources of calcium and phosphate, capable of reacting with the water of the liquid phase to form a cement.

### Detailed description of the invention

This invention deals with the fabrication of a macroporous calcium phosphate foam that can be injectable and that is obtained by foaming the liquid phase of a cement, or a cement paste. The way suggested is the addition of surfactant molecules in the cement, as a way to stabilize the air bubbles in the paste, obtaining a foamed cement. It has been verified that the addition of the surfactant is efficient as foaming agent in the matrix of the cement, producing a high macroporosity with a high degree of interconnection between the macropores.

With the studied formulations, calcium phosphate foam pastes that maintain the macroporosity even after injection can be obtained. Moreover, the surfactant molecules are effective at really low concentrations, close to the critical micelle concentration (CMC). This means that a small quantity of additive is necessary to assure the foaming of the cement paste.

Preferably, the foaming process is performed by agitation or mechanical whipping, according to one of the following protocols:
i) foaming of the liquid fase, followed by mixing with the powder solid phase,
ii) foaming of the aqueous solution containing the surfactant agent, followed by mixing the foam with a paste previously obtained by mixing the solid phase with another aqueous solution that constitutes together with the first one the liquid phase.

The obtained foam sets after mixing, forming a solid structure.

The selection of the surfactant that has been used as foaming agent is a very important point. There exist a big number of molecules that can be used as surfactant agents, with an anionic, cationic or non-ionic character. However, given that the designed material should be injected in the human body and more precisely in the bone tissue, it is necessary to select a biocompatible surfactant.

In this sense, the non-ionic surfactants are of special interest, although there are not the only ones that are suggested. Some of the non-ionic surfactants have good foaming properties and present very good biocompatibility, and are used nowadays in parenteral formulations of drugs. In this sense, non-ionic surfactants are of special utility for this application. Some examples are sorbitan esthers, such as sorbitan monooleate or sorbitan monopalmitate, polyoxysorbitan esthers, such as polyoxyethylene sorbitan monooleate (Tween 80, Polysorbate 80) and polyoxyethylene sorbitan monolaurate (Tween 20). All of them are approved for parenteral formulations of drugs.

Other surfactants that can be used in this application and that are also used for parenteral drugs are some polymeric surfactants, such as those based in ethylene oxide - propylene oxide block polymers (Pluronic F68, Poloxamer 188). Other compounds that are of interest for these applications are some phospholipids, like lecithines, which add biodegradability as a good biological behaviour, and some sucrose esters.

In a preferred embodiment, the non-ionic surfactant is selected from sorbitan monooleate, sorbitan monopalmitate, polyoxyethylene sorbitan monooleate, polyoxyethylene sorbitan monolaurate, polyoxyethylene-polyoxypropylene copolymers, phosphatidylcoline (lecithin), phosphatidylethanolamide, phosphatidylserine, phosphatidylinositol, lisophosphatidylcoline, lisophosphatidylethanolamine, esfingomyeline, sucrose monolaurate, sucrose monooleate, and other sucrose esters.

In a more preferred embodiment the non-ionic surfactant is polyoxyethylene sorbitan monooleate in a concentration in the liquid phase inferior or equal to 20 weight%, preferably inferior or equal to 10 weight %, and preferably inferior or equal to 5 weight %.

The size of the pores can be controlled through different parameters, like the concentration of the added surfactant and the particle size of the cement powder. This is a key aspect to facilitate the angiogenesis and the colonization of the material by newly formed bone.

As a way to increase the interconnectivity of the macropores produced during the foaming process, it is suggested to incorporate some particulate components in the solid phase of the cement. These particles have to be substantially insoluble in the cement but have to dissolve in physiological conditions after the cement foam has set. These particles can be the same as mentioned by Chow et al. (US Pat. No. 5,525,148), Constantz et al. (US Pat. No. 5,820,632) or Hirano et al. (JP 5,023,387).

For the preparation of the foams, any mix and composition of the calcium phosphate cements is possible. The final product of the set cement can vary from dicalcium phosphate dehydrate (Ca/P=1), calcium deficient hydroxyapatite (Ca/P between 1.33 and 1.67), octacalcium phosphate (Ca/P= 1.33), stoichiometric hydroxyapatite (Ca/P= 1.67) or carbonate apatite (Ca/P= 1.7). The main reagents for the solid phase of the cement include a source of calcium and a source of phosphate, which can be present as a unique component or as two or more components. Thus, a unique calcium phosphate can be the principal reagent of the solid phase, as a source of calcium and phosphate. As an alternative, two or more components can be present in the solid phase of the cement and each of them can contain calcium, phosphate or calcium and phosphate. Some of the interesting calcium phosphates can be selected between the following: tetratracalcium phosphate, dicalcium phosphate anhydrous, dicalcium phosphate dihydrate, alfa-tricalcium phosphate, beta-tricalcium phosphate, monocalcium phosphate monohydrate, hydroxyapatite, calcium deficient hydroxyapatite, fluorapatite, amorphous calcium phosphate, calcium- sodium- and potassium- phosphate, calcium- and sodium- phosphate, calcium- and potassium- phosphate, and calcium pyrophosphate. Other interesting components as calcium sources are the following: calcium carbonate, calcium sulphate, sulfato cálcico hemihidratado, calcium oxide or calcium hydroxide. Among the sources of calcium, all the soluble phosphates and the phosphoric acid can be mentioned. The reagents should set after mixing with the liquid phase, which is an aqueous solution. This requires a precise selection of the calcium phosphates, which form part of the powder phase, their proportions and their characteristics, as it is shown in different patents (for example, Brown y Chow U.S. Pat No. RE 33,161, U.S. Pat. No. RE 33,221, Chow and Takagi US Pat. No. 5,525,148, Constantz U.S. Pat. No. 4,880,610, U.S. Pat. 5,820,632, US Pat. No. 6,375,935, among others).

In another preferred embodiment, the solid phase comprises alpha tricalcium phosphate, with a medium particle size inferior to 100 micrometers, preferrably inferior to 50 micrometers, preferrably inferior to 30 micrometers, preferrably inferior to 15 micrometers.

The solid phase can also contain additives which act as seeds, such as precipitated tricalcium phosphate, which facilitate the nucleation of the phase which precipitate in the cement. The amount usually is inferior to 10 weight % and preferably inferior to 5 weight % with regard to the solid phase. The solid phase can also contain granules of a biodegradable substance to increase the interconnectivity between the pores and the foam. The dimensions of these granules should be between 10 and 500µm and can be either of polymeric or inorganic nature, such as calcium salts or soluble glasses.

The liquid phase comprises an aqueous solution that can incorporate one or more phosphate, carbonate or silicate ions in dissolution, which can act as accelerants or retarders of the setting reaction, and which can be obtained by dissolution of different compounds. Among the accelerants, Na₂HPO₄, NaH₂PO₄, KH₂PO₄, K₂HPO₄, can be mentioned. Preferably, the accelerant of the setting reaction is Na₂HPO₄ in a concentration between 0.1 and 10 weight %, and more preferably between 1 and 5 weight % of the liquid phase. The liquid phase can also comprise one or more oligomeric compounds or polymers either dissolved or in suspension in the liquid phase of the cement, such as sodium alginate, hydroxypropyl methyl cellulose, hydroxyethyl cellulose, hydroxypropyl cellulose, methyl cellulose, hydroxyethyl starch, soluble starch, cyclodextrin, dextran sulphate, polyvinylpyrrolidone, quitosan, and hyaluronic acid, to increase the injectability and the cohesion of the cement paste, avoiding the disintegration of the same as it is submerged in the physiological medium, as mentioned in Driessens et al. U.S. Pat. No. 6,206,957.

Preferably the liquid phase comprises sodium alginate, as a cohesion promoter agent, in a concentration between 0.05 and 10 weight %, more preferably between 0.1 and 5 weight %.

In addition, to obtain the foam the liquid phase contains at least a biocompatible surfactant, which acts as foaming agent.

In a preferred embodiment, the relation between the liquid phase and the solid phase is superior to 0.25 ml/g, preferably superior to 0.32 ml/g, and preferably superior to 0.35 ml/g.

The calcium phosphate foam can also incorporate one or more biologically active agents such as growth factors and/ or anticancer substances and/or antibiotics and/or antioxidants.

Once foamed, the obtained mixture can be injected using a syringe with a diameter between 1 and 15 mm, after a time inferior to 60 minutes after mixing has elapsed, preferably inferior to 30 minutes, preferably inferior to 15, preferably inferior to 10, and preferably equal or inferior to 5 minutes.

The foam according to the present invention, after setting, allows to obtain a solid structure of a total porosity comprised between 25 and 90 vol % and a macroporosity comprised between 2 and 80 vol %. Said solid structure comprises macropores having a diameter comprised between 20 and 800 µm, preferably between 100 and 700 µm, which constitute a macroporosity superimposed to the intrinsic porosity of the set cement, which is in the micro and/or nanoscopic level.

### Description of the drawings

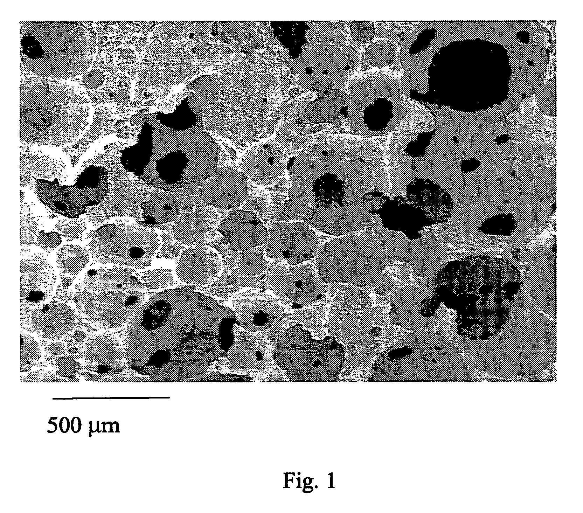

Fig. 1. As an illustrative example, a micrograph of a section of the calcium phosphate foam obtained by scanning electronic microscopy is shown. The calcium phosphate foam is obtained by the addition of a 5 weight% of a biocompatible non-ionic surfactant to the liquid phase of an alfa-tricalcium phosphate cement, using a liquid to powder ratio of 0.45 ml/g.

### Detailed description of particular embodiments

Next, the invention will be described with more details in some examples, although it must be understood that the invention is not restricted only to the compositions specified in the examples.

### Example 1

For the foam preparation, a solution containing a 10 weight % of the non- ionic surfactant, Pluronic F68 (Polyoxyethylene-polyoxypropylene Copolymer), was prepared either in bidistilled water, or in an aqueous solution of 2.5 weight % of Na₂HPO₄. 2 ml of the solution were used to prepare the foam via mechanical agitation. Once prepared, the foam was mixed with the powder phase of the cement, with a liquid to powder relation of 0.38 ml/g. The powder phase consisted of 5.155g of alfa-Tricalcium phosphate (α-TCP) with a medium size of 6.2µm and 0.105g of precipitated tricalcium phosphate (Merck, ref N°. 2143) which had been mixed previously. Another foam was prepared using a 5 weight% of surfactant in the liquid phase. All the series were repeated using α-TCP with a smaller particle size, medium size of 3.6µm. As reference, some cements without surfactant agent were prepared. The initial setting time was measured with the Gillmore needles method. All the series were prepared in the form of cylinders with a diameter of 6 mm and a height of 12 mm. The foam was injected in Teflon moulds and immerged in Ringer's solution (aqueous solution with 0.9% NaCl) at 37° C for 64 hours to simulate physiological conditions. The phase composition was determined via X-ray diffraction. The apparent density of the samples was measured by immersion in mercury and in comparison with a cement without foaming, the macroporosity and the microporosity of the foam were calculated. The microstructure was examined on fracture surfaces of the set foams via scanning electron microscopy.

For all series prepared, it was observed that, when mixing the foam with the cement powder, a macroporous paste was obtained. Once submerged in the Ringer's solution, the foamed cement pastes maintained the cohesion, giving as a result after setting solid foams of hydroxyapatite with different degrees of macroporosity. The setting times, the total porosity and macroporosity are presented in Table 1.

**TABLE 1. Initial setting time, total porosity and macroporosity of the different foams prepared. The standard deviation is given between brackets. N.M. not measured.**

| Pluronic F68 (weight%) | Na₂HPO₄ (weight%) | Powder size | Setting time (min) | Total porosity (%) | Macroporosity (%) |
|---|---|---|---|---|---|
| 10 | 0 | Fine | 38 | 57.7 (1.0) | 15.3 (1.9) |
| 10 | 0 | Coarse | 42 | 63.0 (3.4) | 27.9 (6.7) |
| 10 | 2,5 | Fine | 12 | 58.6 (0.5) | 15.8 (0.9) |
| 10 | 2,5 | Coarse | 15 | 57.1 (1.3) | 13.4 (2.6) |
| 5 | 0 | Fine | 42 | 54.8 (1.8) | 9.5 (3.2) |
| 5 | 0 | Coarse | 47 | 60.6 (3.2) | 23.1 (6.2) |
| 5 | 2,5 | Fine | 13 | 55.9 (1.1) | 10.4 (2.2) |
| 5 | 2,5 | Coarse | 14 | 59.7 (0.9) | 18.8 (1.7) |
| 0 | 0 | Fine | N.M. | 50.1 (0.1) | - |
| 0 | 0 | Coarse | M.M. | 48.8 (1.3) | - |
| 0 | 2,5 | Fine | N.M. | 50.8 (0.7) | - |
| 0 | 2,5 | Coarse | N.M. | 50.4 (0.9) | - |

It was observed that within the range studied, the surfactant concentration, did not have any effect on the macroporosity obtained. The addition of Na₂HPO₄ reduced the setting times of the foams, although it also produced a decrease of the total macroporosity. In general, more porous foams were obtained when the coarse α-TCP powder was used.

### Example 2

For the preparation of the foam, an aqueous solution containing 2.5 weight% Na₂HPO₄ and 20 weight% non- ionic surfactant Tween 80 (Polyoxyethylene sorbitan monooleate) were used. 2 ml of the solution were foamed via mechanical agitation. Once the foam was prepared, it was mixed with the cement powder in a liquid to powder ratio of 0.32 ml/g. The powder phase consisted of 6.127g of alpha tricalcium phosphate (α-TCP) and 0.125g of precipitated tricalcium phosphate (Merck catalogue N°. 2143), which were previously mixed. Other foams were also prepared using different surfactant concentrations in the liquid phase (10 and 5 weight %), and different liquid to powder ratios (0.38 and 0.45 ml/g). As reference, a cement without surfactant agent in the liquid phase was prepared. For all series cylinders with a diameter of 6 mm and a height of 12 mm were prepared by injecting the foam in Teflon moulds. These moulds were immersed in Ringer's solution (aqueous solution with 0.9% NaCl) at 37° C for 64 hours. The phase composition was determined via X-ray diffraction. The apparent density of the samples was measured by immersion in mercury and in comparison with a cement without foaming, the macroporosity and the microporosity of the foam were calculated. The microstructure was examined in fracture surfaces of the set foams by scanning electron microscopy.

For all the series prepared it was observed that, when mixing the foam with the cement powder, a macroporous paste was obtained. Once immersed in the Ringer's solution, the foamed cement pastes maintained the cohesion, giving as a result after setting solid foams of hydroxyapatite with different degrees of macroporosity, between 51 and 69%, as is shown in Table 2.

**TABLE 2. Total porosity, macroporosity and compressive strength for the different foams. The standard deviation is given between brackets. N.M.: not measured.**

| L/P (ml/g) | Tween 80 (% peso) | Total porosity (%) | Macroporosity (%) | Compressive strength (MPa) |
|---|---|---|---|---|
| 0.32 | 20 | 51.2 (0.4) | 10.1 (0.8) | 10.40 (±1.99) |
| | 10 | 51.2 (0.5) | 10.1 (1.0) | 18.14 (±4.27) |
| | 5 | 51.4 (0.2) | 10.5 (0.4) | 16.46 (±3.68) |
| | 0 | 45.7 (0.5) | - | N.M. |
| 0.38 | 20 | 55.1 (1.2) | 9.3 (2.5) | 14.19 (±1.85) |
| | 10 | 54.9 (1.0) | 9.1 (1.7) | 7.51 (±1.60) |
| | 5 | 59.0 (1.8) | 17.2 (3.7) | 5.97 (±0.99) |
| | 0 | 50.4 (0.5) | - | N.M. |
| 0.45 | 20 | 64.1 (0.7) | 19.5 (1.6) | 2.99 (±0.89) |
| | 10 | 67.3 (±1.4) | 26.8 (3.0) | 1.36 (±0.24) |
| | 5 | 68.9 (±0.5) | 30.3 (1.0) | 1.80 (±0.63) |
| | 0 | 55.4 (0.6) | - | N.M. |

The macroporosity varied between 10 and 30%, the size of the macropores being between 70 and 700 micrometers. An increase in the macroporosity as well as in the pore size was observed when the liquid to powder ratio was increased and when the surfactant concentration in the liquid phase was decreased. The most porous samples also showed a high interconnectivity between the pores. In all cases, besides the macroporosity produced during foaming, the samples presented a high intrinsic micro/nanoporosity in the calcium phosphate cement.

### Example 3

Foams were prepared, using the surfactant Tween 80, according to the protocol mentioned in Example 2, with a concentration of 5 and 20 weight% and a liquid to powder relation of 0.40 and 0.45 ml/g. The injectability was measured using a mechanical testing machine. The foamed paste was placed in a 5 ml syringe, with an injection hole of 2 mm in diameter. The injectability was calculated as the mass percentage of the extruded paste when the piston of the syringe was compressed with a velocity of 15 mm/min and up to a maximum force of 100N. This test was performed 5 min after mixing of the powder with the foam. The results obtained are summarized in Table 3.

**TABLE 3. Injectability of the different foams in paste form.**

| Tween 80 (weight%) | L/P (ml/g) | Inyectability (%) |
|---|---|---|
| 5 | 40 | 48.2 |
| 5 | 45 | 78.4 |
| 20 | 40 | 35.9 |
| 20 | 45 | 65.8 |

## Claims

1. Process for the preparation of a self setting calcium phosphate foam, **characterized in that** it comprises the mixture of two phases:
a) a liquid phase consisting of one or two aqueous solutions, one of them comprising at least one biocompatible surfactant agent responsible for the foam creation, and
b) a solid phase in powder form, comprising one or more compounds as sources of calcium and phosphate, capable of reacting with the water of the liquid phase to form a cement,
wherein the liquid phase is mixed after being submitted to a foaming process, and wherein the relation between the liquid phase and the solid phase is superior to 0.25 ml/g.

2. The process according to claim 1, wherein the relation between the liquid phase and the solid phase is superior to 0.32 ml/g.

3. The process according to claim 1, wherein the relation between the liquid phase and the solid phase is superior to 0.35 ml/g.

4. Process according to any one of claims 1-3, **characterized in that** the foaming process is performed by agitation or mechanical whipping, according to one of the following protocols:
i) foaming of the liquid phase, followed by mixing with the powder solid phase, or
ii) foaming of the aqueous solution containing the surfactant agent, followed by mixing the foam with a paste previously obtained by mixing the solid phase with another aqueous solution that constitutes together with the first one the liquid phase,
wherein the obtained foam sets after mixing, forming a solid structure.

5. Process according to any one of claims 1-4, **characterized in that** the surfactant agent is a non-ionic surfactant.

6. Process according to claim 5, **characterized in that** the non-ionic surfactant is selected from sorbitan monooleate, sorbitan monopalmitate, polyoxyethylene sorbitan monooleate, polyoxyethylene sorbitan monolaurate, polyoxyethylene-polyoxypropylene copolymers, phosphatidylcoline, phosphatidylethanolamide, phosphatidylserine, phosphatidylinositol, lisophosphatidylcoline, lisophosphatidylethanolamine, esfingomyeline, sucrose monolaurate, sucrose monooleate, and other sucrose esters.

7. Process according to claim 6, **characterized in that** the non-ionic surfactant is polyoxyethylene sorbitan monooleate in a concentration in the liquid phase inferior or equal to 20 weight%.

8. Process according to claim 7, wherein the concentration in the liquid phase is inferior or equal to 10 weight %.

9. Process according to claim 7, wherein the concentration in the liquid phase is inferior or equal to 5 weight %.

10. Process according to any one of claims 1 to 9, **characterized in that** the liquid phase further comprises an accelerant of the setting reaction, selected from Na₂HPO₄, NaH₂PO₄ KH₂PO₄, and K₂HPO₄.

11. Process according to claim 10, **characterized in that** said accelerant of the setting reaction is Na₂HPO₄ in a concentration between 0.1 and 10 weight % of the liquid phase.

12. Process according to claim 11, wherein the concentration of Na₂HPO₄ is between 1 and 5 weight % of the liquid phase.

13. Process according to any one of claims 1 to 12, **characterized in that** the liquid phase further comprises at least a cohesion promoter agent for the cement, selected from sodium alginate, hydroxypropyl methyl cellulose, hydroxyethyl cellulose, hydroxypropyl cellulose, methyl cellulose, hydroxyethyl starch, soluble starch, cyclodextrin, dextran sulphate, polyvinylpyrrolidone, quitosan, and hyaluronic acid.

14. Process according to claim 13, **characterized in that** said cohesion promoter is sodium alginate with a concentration between 0.05 and 10 weight %.

15. Process according to claim 14, wherein the concentration of sodium alginate is between 0.1 and 5 weight %.

16. Process according to any one of claims 1 to 15, **characterized in that** the solid phase comprises as a calcium and phosphate source:
i) at least a compound of calcium and phosphate selected from tetracalcium phosphate, dicalcium phosphate anhydride, dicalcium phosphate dihydrate, alpha tricalcium phosphate, beta tricalcium phosphate, monocalcium phosphate monohydrate, hydroxyapatite, calcium deficient hydroxyapatite, fluorapatite, amorphous calcium phosphate, calcium- sodium- and potassium- phosphate, calcium- and sodium- phosphate, calcium- and potassium- phosphate, and calcium pyrophosphate; or alternatively
ii) at least a compound of calcium selected from calcium carbonate, calcium sulphate, calcium sulphate hemi hydrate, calcium oxide, and calcium hydroxide, and at least a compound of phosphate selected from phosphoric acid and all the soluble phosphates; or alternatively
iii) a mixture of at least a compound defined in option i) and at least a compound defined in option ii).

17. Process according to claim 16, **characterized in that** the solid phase comprises alpha tricalcium phosphate with a medium particle size inferior to 100 micrometers.

18. Process according to claim 17, wherein the medium particle size is inferior to 50 micrometers.

19. Process according to claim 17, wherein the medium particle size is inferior to 30 micrometers.

20. Process according to claim 17, wherein the medium particle size is inferior to 15 micrometers.

21. Process according to any one of claims 1 to 20, **characterized in that** the solid phase further comprises precipitated tricalcium phosphate as an additive acting as seed material, in a quantity inferior to 10 weight % with regard to the solid phase.

22. Process according to claim 21, wherein the amount of precipitated tricalcium phosphate is inferior to 5 weight % with regard to the solid phase.

23. Process according to any one of claims 1 to 22, **characterized in that** the solid phase further comprises granules of a biodegradable substance to increase the interconnectivity between the pores and the foam, the dimensions of these granules being comprised between 10 and 500 micrometers.

24. Process according to claim 23, wherein the granules are of polymeric nature.

25. Process according to claim 23, wherein the granules are of calcium salts.

26. Process according to claim 23, wherein the granules are of soluble glasses.

27. Process according to any one of claims 1 to 26, **characterized in that** the liquid phase further comprises one or more agents to improve the injectability.

28. Self setting calcium phosphate foam obtainable by the process of any one of claims 1 to 27.

29. Self setting calcium phosphate foam according to 28, **characterized in that** it comprises one or more biologically active agents.

30. Self setting calcium phosphate foam according to claim 29, wherein the biologically active agents are selected from growth factors, anticancerogenic substances, antibiotics, and antioxidants, or a mixture thereof.

31. Solid structure obtainable by setting of the foam according to any one of claims 28-30, having a total porosity comprised between 25 and 90 vol % and a macroporosity comprised between 2 and 80 vol %.

32. Solid structure according to claim 33, comprising macropores having a diameter comprised between 20 and 800 µm.

33. Solid structure according to claim 34, wherein the diameter of the macropores is comprised between 100 and 700 µm.

34. Use of the foam according to any one of claims 28 to 30 for the manufacture of a solid structure for bone regeneration, both in putty or preset state.

35. Use of the foam according to any one of claims 28 to 30 for the manufacture of a scaffold or substrate for tissue engineering.

## Patentansprüche

1. Verfahren zur Herstellung eines selbstaushärtenden Calciumphosphat-Schaums **dadurch gekennzeichnet, dass** es die Mischung aus zwei Phasen umfasst:
a) einer flüssigen Phase bestehend aus einer oder zwei wässrigen Lösungen, von denen eine mindestens ein biokompatibles Tensid umfasst, das die Schaumerzeugung veranlasst, und
b) einer festen Phase in Form eines Pulvers, umfassend eine oder mehrere Verbindungen als Calcium- und Phosphatquellen, die mit dem in der flüssigen Phase vorhandenen Wasser reagieren können, um Beton zu erzeugen,
wobei die flüssige Phase gemischt wird, nachdem sie zum Schäumen gebracht worden ist, und wobei das Verhältnis der flüssigen Phase zu der festen Phase bei über 0,25 ml/g liegt.

2. Verfahren nach Anspruch 1, wobei das Verhältnis der flüssigen Phase zu der festen Phase bei über 0,32 ml/g liegt.

3. Verfahren nach Anspruch 1, wobei das Verhältnis der flüssigen Phase zu der festen Phase bei über 0,35 ml/g liegt.

4. Verfahren nach einem der Ansprüche 1-3 **dadurch gekennzeichnet, dass** das Schäumen durch Rühren oder mechanisches Schlagen nach einem der folgenden Vorgehensweisen durchgeführt wird:
i) Schäumen der flüssigen Phase, gefolgt vom Abmischen mit der in Form eines Pulvers vorhandenen festen Phase, oder
ii) Schäumen der wässrigen Lösung umfassend das Tensid, gefolgt vom Abmischen des Schaums mit einer Paste, die im Vorfeld durch das Abmischen der festen Phase mit einer anderen wässrigen Lösung erhalten worden ist, die mit der ersten Lösung die flüssige Phase ausmacht,
wobei der erhaltene Schaum nach dem Abmischen aushärtet und somit eine feste Struktur bildet.

5. Verfahren nach einem der Ansprüche 1-4 **dadurch gekennzeichnet, dass** das Tensid ein nichtionisches Tensid ist.

6. Verfahren nach Anspruch 5 **dadurch gekennzeichnet, dass** das nichtionische Tensid aus Sorbitanmonooleat, Sorbitanmonopalmitat, Polyoxyethylensorbitanmonooleat, Polyoxyethylensorbitanmonolaurat, Copolymeren aus Polyoxyethylen- Polyoxypropylen, Phosphatidylcholin, Phosphatidylethanolamid, Phosphatidylserin, Phosphatidylinositol, Lisophosphatidylcholin, Lisophosphatidylethanolamin, Sphingomyelin, Saccharosemonolaurat, Saccharosemonooleat, und anderen Estern aus Saccharose ausgewählt ist.

7. Verfahren nach Anspruch 6 **dadurch gekennzeichnet, dass** das nichtionische Tensid Polyoxyethylensorbitanmonooleat in einer Konzentration innerhalb der flüssigen Phase ist, die bei 20 Gew.-% oder darunter liegt.

8. Verfahren nach Anspruch 7, wobei die Konzentration innerhalb der flüssigen Phase bei 10 Gew.-% oder darunter liegt.

9. Verfahren nach Anspruch 7, wobei die Konzentration innerhalb der flüssigen Phase bei 5 Gew.-% oder darunter liegt.

10. Verfahren nach einem der Ansprüche 1 bis 9 **dadurch gekennzeichnet, dass** die flüssige Phase weiterhin einen Reaktionsbeschleuniger für die Aushärtungsreaktion umfasst, der aus Na₂HPO₄, NaH₂PO₄, KH₂PO₄, und K₂HPO₄ ausgewählt ist.

11. Verfahren nach Anspruch 10 **dadurch gekennzeichnet, dass** der Reaktionsbeschleuniger für die Aushärtungsreaktion Na₂HPO₄ in einer Konzentration ist, die bei zwischen 0,1 und 10 Gew.-% bezogen auf die flüssige Phase liegt.

12. Verfahren nach Anspruch 11, wobei die Konzentration vom Na₂HPO₄ bei zwischen 1 und 5 Gew.-% bezogen auf die flüssige Phase liegt.

13. Verfahren nach einem der Ansprüche 1 bis 12 **dadurch gekennzeichnet, dass** die flüssige Phase weiterhin mindestens einen Haftvermittler für den Beton umfasst, der aus Natriumalginat, Hydroxypropylmethylcellulose, Hydroxyethylcellulose, Hydroxypropylcellulose, Methylcellulose, Hydroxyethylstärke, löslicher Stärke, Cyclodextrin, Dextransulfat, Polyvinylpyrrolidon, Chitosan, und Hyaluronsäure ausgewählt ist.

14. Verfahren nach Anspruch 13 **dadurch gekennzeichnet, dass** der Haftvermittler Natriumalginat in einer Konzentration zwischen 0,05 und 10 Gew.-% vorhanden ist.

15. Verfahren nach Anspruch 14, wobei die Konzentration vom Natriumalginat von 0,1 bis 5 Gew.-% reicht.

16. Verfahren nach einem der Ansprüche 1 bis 15 **dadurch gekennzeichnet, dass** die feste Phase als Calcium- und Phosphatquelle folgendes umfasst:
i) mindestens eine calcium- und phosphathaltige Verbindung, die ausgewählt aus Tetracalciumphosphat, Dicalciumphosphat-Anhydrid, Dicalciumphosphat-Dihydrat, alpha-Tricalciumphosphat, beta-Tricalciumphosphat, Monocalciumphosphat- Monohydrat, Hydroxyapatit, calciumarmem Hydroxyapatit, Fluorapatit, amorphem Calciumphosphat, Calcium- Natrium- und Kaliumphosphat, Calcium- und Natriumphosphat, Calcium- und Kaliumphosphat, und Calciumpyrophosphat ist; oder wahlweise
ii) mindestens eine calciumhaltige Verbindung, die aus Calciumcarbonat, Calciumsulfat, Calciumsulfat-Hemihydrat, Calciumoxid, und Calciumhydroxid ausgewählt ist, und mindestens eine phosphathaltige Verbindung, die aus Phosphorsäure und allen löslichen Phosphaten ausgewählt ist; oder wahlweise
iii) eine Mischung aus mindestens einer Verbindung entsprechend der Definition in Alternative i) und mindestens eine Verbindung entsprechend der Definition in Alternative ii).

17. Verfahren nach Anspruch 16 **dadurch gekennzeichnet, dass** die feste Phase alpha-Tricalciumphosphat mit einer durchschnittlichen Partikelgröße unter 100 Mikrometern umfasst.

18. Verfahren nach Anspruch 17, wobei die durchschnittliche Partikelgröße bei unter 50 Mikrometern liegt.

19. Verfahren nach Anspruch 17, wobei die durchschnittliche Partikelgröße bei unter 30 Mikrometern liegt.

20. Verfahren nach Anspruch 17, wobei die durchschnittliche Partikelgröße bei unter 15 Mikrometern liegt.

21. Verfahren nach einem der Ansprüche 1 bis 20 **dadurch gekennzeichnet, dass** die feste Phase weiterhin ausgefälltes Tricalciumphosphat in einem Verhältnis unter 10 Gew.-% bezogen auf die feste Phase als Zusatzstoff umfasst, der als Saatmaterial dient.

22. Verfahren nach Anspruch 21, wobei das Verhältnis vom ausgefällten Tricalciumphosphat bei unter 5 Gew.-% bezogen auf die feste Phase liegt.

23. Verfahren nach einem der Ansprüche 1 bis 22 **dadurch gekennzeichnet, dass** die feste Phase weiterhin Granulat eines biologisch abbaubaren Stoffs zur Erhöhung der Verbundenheit zwischen den Poren und dem Schaum umfasst, wobei die Partikelgröße dieses Granulats bei zwischen 10 und 500 Mikrometern liegt.

24. Verfahren nach Anspruch 23, wobei das Granulat ein Polymergranulat ist.

25. Verfahren nach Anspruch 23, wobei das Granulat aus Calciumsalzen besteht.

26. Verfahren nach Anspruch 23, wobei das Granulat aus löslichen Kristallen besteht.

27. Verfahren nach einem der Ansprüche 1 bis 26 **dadurch gekennzeichnet, dass** die flüssige Phase weiterhin einen oder mehrere Stoffe umfasst, welche die Injizierbarkeit verbessern.

28. Selbstaushärtender Calciumphosphat-Schaum herstellbar nach einem Verfahren nach einem der Ansprüche 1 bis 27.

29. Selbstaushärtender Calciumphosphat-Schaum nach Anspruch 28 **dadurch gekennzeichnet, dass** er einen oder mehrere Wirkstoffe umfasst.

30. Selbstaushärtender Calciumphosphat-Schaum nach Anspruch 29, wobei die Wirkstoffe aus Wachstumsfaktoren, antikanzerogenen Stoffen, Antibiotika, und Antioxidationsmitteln, oder einer Mischung davon ausgewählt sind.

31. Feste Struktur herstellbar durch Aushärtung des Schaums nach einem der Ansprüche 28-30, die eine gesamte Porosität im Bereich zwischen 25 und 90 Vol.-% und eine Makroporosität im Bereich zwischen 2 und 80 Vol.-% aufweist.

32. Feste Struktur nach Anspruch 33, die Makroporen umfasst, die einen Durchmesser im Größenbereich zwischen 20 und 800 µm aufweisen.

33. Feste Struktur nach Anspruch 34, wobei der Durchmesser der Makroporen im Größenbereich zwischen 100 und 700 µm liegt.

34. Verwendung eines Schaums nach einem der Ansprüche 28 bis 30 zur Herstellung einer festen Struktur zur Knochenregeneration, als Kitt oder in einem noch nicht ausgehärteten Zustand.

35. Verwendung des Schaums nach einem der Ansprüche 28 bis 30 zur Herstellung eines Gerüsts oder eines Substrats zur Gewebezüchtung.

## Revendications

1. Procédé de préparation d'une mousse auto-durcissante de phosphate de calcium **caractérisée en ce qu'**elle comprend un mélange de deux phases :
a) une phase liquide consistant en une ou deux solutions aqueuses, comprenant l'une d'eux au moins un agent surfactant biocompatible qui est responsable de la formation de la mousse, et
b) une phase solide en forme d'une poudre, comprenant un ou plusieurs composés en tant que sources de calcium et de phosphate, qui est capable de réagir avec l'eau de la phase liquide pour former un ciment,
dans lequel la phase liquide est mélangée après subir un processus de moussage, et dans lequel le rapport de la phase liquide à la phase solide est supérieur à 0,25 ml/g.

2. Procédé selon la revendication 1, dans lequel le rapport de la phase liquide à la phase solide est supérieur à 0,32 ml/g.

3. Procédé selon la revendication 1, dans lequel le rapport de la phase liquide à la phase solide est supérieur à 0,35 ml/g.

4. Procédé selon l'une quelconque des revendications 1 à 3 **caractérisé en ce que** le processus de moussage est effectué par agitation ou battement mécanique, selon l'une quelconque des processus suivants:
i) moussage de la phase liquide suivi du mélange avec la poudre de la phase solide, ou,
ii) moussage de la solution aqueuse contenant l'agent surfactant, suivi du mélange de la mousse avec une pâte précédemment obtenue par mélange de la phase solide avec une autre solution aqueuse constituant, avec la première, la phase liquide,
dans lequel la mousse obtenue se durcit après avoir être mélangée, en formant une structure solide.

5. Procédé selon l'une quelconque des revendications 1-4 **caractérisé en ce que** l'agent surfactant est un surfactant non ionique.

6. Procédé selon la revendication 5 **caractérisé en ce que** le surfactant non ionique est choisi parmi le monooléate de sorbitan, le monopalmitate de sorbitan, le monooléate de polyoxyéthylène sorbitan, le monolaurate de polyoxyéthylène sorbitan, des copolymères de polyoxyéthylène-polyoxypropylène, la phosphatidylcholine, la phosphatidyléthanolamide, la phosphatidylsérine, le phosphatidylinositol, la lisophosphatidylcholine, la lisophosphatidyléthanolamine, la sphingomyéline, le monolaurate de sucrose, le monooléate de sucrose, et d'autres esters de sucrose.

7. Procédé selon la revendication 6, **caractérisé en ce que** le surfactant non ionique est le monooléate de polyoxyéthylène sorbitan dans une concentration dans la phase liquide inférieure ou égale à 20% en poids.

8. Procédé selon la revendication 7, dans lequel la concentration dans la phase liquide est inférieure ou égale à 10% en poids.

9. Procédé selon la revendication 7, dans lequel la concentration dans la phase liquide est inférieure ou égale à 5% en poids.

10. Procédé selon l'une quelconque des revendications 1 à 9 **caractérisé en ce que** la phase liquide comprend en outre un accélérateur de la réaction de durcissement, choisi parmi le Na₂HPO₄, NaH₂PO₄, KH₂PO₄, et le K₂HPO₄.

11. Procédé selon la revendication 10, **caractérisé en ce que** ledit accélérateur de la réaction de durcissement est le Na₂HPO₄ dans une concentration comprise entre 0,1 et 10% en poids de la phase liquide.

12. Procédé selon la revendication 11, dans lequel la concentration de Na₂HPO₄ est comprise entre 1 % et 5% en poids de la phase liquide.

13. Procédé selon l'une quelconque des revendications 1 à 12 **caractérisé en ce que** la phase liquide comprend en outre un agent promoteur de la cohésion pour le ciment, choisi parmi l'alginate de sodium, l'hydroxypropylméthylcéllulose, l'hydroxyéthylcéllulose, l'hydroxypropylcéllulose, la méthylcéllulose, un hydroxyéthylamidon, de l'amidon soluble, une cyclodextrine, le sulfate de dextrane, la polyvinylpyrrolidone, le chitosane, et l'acide hyaluronique.

14. Procédé selon la revendication 13, **caractérisé en ce que** ledit promoteur de la cohésion est l'alginate de sodium avec une concentration comprise entre 0,05 et 10% en poids.

15. Procédé selon la revendication 14, dans lequel la concentration d'alginate de sodium est comprise entre 0,1 et 5% en poids.

16. Procédé selon l'une quelconque des revendications 1 à 15 **caractérisé en ce que** la phase solide comprend en tant que source de calcium et de phosphate:
i) au moins un composé de calcium et de phosphate choisi parmi le phosphate de tétracalcium, le phosphate de dicalcium anhydre, le phosphate de dicalcium dihydraté, le phosphate d'alpha-tricalcium, le phosphate de bêta-tricalcium, le phosphate de monocalcium monohydraté, l'hydroxyapatite, l'hydroxyapatite déficiente en calcium, la fluoroapatite, le phosphate de calcium amorphe, le phosphate de calcium/sodium/potassium, le phosphate de calcium/sodium, le phosphate de calcium/potassium, et le pyrophosphate de calcium ; ou, facultativement
ii) au moins un composé de calcium choisi parmi le carbonate de calcium, le sulfate de calcium, le sulfate de calcium hémi-hydrate, l'oxyde de calcium, et l'hydroxyde de calcium, et, au moins, un composé de phosphate choisi parmi l'acide phosphorique et tous les phosphates solubles ; ou, facultativement
iii) un mélange d'au moins un composé tel que défini dans l'option i) et au moins un composé tel que défini dans l'option ii).

17. Procédé selon la revendication 16, **caractérisé en ce que** la phase solide comprend du phosphate d'alpha-tricalcium ayant une taille de particules moyenne inférieure à 100 micromètres.

18. Procédé selon la revendication 17, dans lequel la taille de particules moyenne est inférieure à 50 micromètres.

19. Procédé selon la revendication 17, dans lequel la taille de particules moyenne est inférieure à 30 micromètres.

20. Procédé selon la revendication 17, dans lequel la taille de particules moyenne est inférieure à 15 micromètres.

21. Procédé selon l'une quelconque des revendications 1 à 20 **caractérisé en ce que** la phase solide comprend en outre du phosphate de tricalcium précipité en tant qu'additif agissant comme matériau de semence dans une quantité inférieure à 10% en poids par rapport à la phase solide.

22. Procédé selon la revendication 21, dans lequel la quantité de phosphate de tricalcium précipité est inférieure à 5% en poids para rapport à la phase solide.

23. Procédé selon l'une quelconque des revendications 1 à 22 **caractérisé en ce que** la phase solide comprend en outre des granules de substance biodégradable pour augmenter l'interconnectivité entre les pores et la mousse, étant les dimensions de ces granules comprises entre 10 et 500 micromètres.

24. Procédé selon la revendication 23, dans lequel les granules sont de nature polymérique.

25. Procédé selon la revendication 23, dans lequel les granules sont granules de sels de calcium.

26. Procédé selon la revendication 23, dans lequel les granules sont granules de cristaux solubles.

27. Procédé selon l'une quelconque des revendications 1 à 26 **caractérisé en ce que** la phase liquide comprend en outre un ou plusieurs agents pour améliorer la capacité d'injection.

28. Mousse de phosphate de calcium auto-durcissante qui peut être obtenue par le biais d'un procédé selon l'une quelconque des revendications 1 à 27.

29. Mousse de phosphate de calcium auto-durcissante selon la revendication 28 **caractérisée en ce qu'**elle comprend un ou plusieurs agents biologiquement actifs.

30. Mousse de phosphate de calcium auto-durcissante selon la revendication 29, dans laquelle les agents biologiquement actifs sont choisis parmi des facteurs de croissance, des substances anti-cancérogènes, des antibiotiques, et des antioxydants, ou un mélange de ceux-ci.

31. Structure solide qui peut être obtenue par durcissement de la mousse selon l'une quelconque des revendications 28 à 30, ayant une porosité totale comprise entre 25 et 90% en volume et une macroporosité comprise entre 2 et 80% en volume.

32. Structure solide selon la revendication 33, comprenant des macropores ayant un diamètre compris entre 20 et 800 µm.

33. Structure solide selon la revendication 34, dans laquelle le diamètre des macropores est compris entre 100 et 700 µm.

34. Utilisation d'une mousse selon l'une quelconque des revendications 28 à 30 pour la manufacture d'une structure solide pour la régénération osseuse, sous forme d'un mastic ou dans l'état pré-durci.

35. Utilisation de la mousse selon l'une quelconque des revendications 28 à 30 pour la manufacture d'un échafaudage ou substrat pour ingénierie tissulaire.
